(19) Europäisches Patentamt
European Patent Office
Office européen des brevets

(11) **EP 3 771 470 A1**

(12) **EUROPEAN PATENT APPLICATION**

(43) Date of publication:
**03.02.2021 Bulletin 2021/05**

(51) Int Cl.:
**A61L 15/28** (2006.01)       **A61L 15/60** (2006.01)
**A61L 26/00** (2006.01)

(21) Application number: **19397524.0**

(22) Date of filing: **29.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **UPM-Kymmene Corporation**
**00100 Helsinki (FI)**

(72) Inventors:
• **Nuopponen, Markus**
  **00220 Helsinki (FI)**

• **Luukko, Kari**
  **02660 Helsinki (FI)**
• **Yliperttula, Marjo**
  **02330 Espoo (FI)**
• **Koivuniemi, Raili**
  **01690 Vantaa (FI)**
• **Kiiskinen, Jasmi**
  **01350 Vantaa (FI)**

(74) Representative: **Berggren Oy, Tampere**
**Visiokatu 1**
**33720 Tampere (FI)**

(54) **MEDICAL HYDROGEL COMPRISING NANOFIBRILLAR CELLULOSE, TAILORED WOUND DRESSING, AND METHODS FOR PREPARING THEREOF**

(57)   The present application relates to a medical hydrogel comprising nanofibrillar cellulose, wherein the content of nanofibrillar cellulose in the hydrogel is in the range of 1-3.5% (w/w), and the nanofibrillar cellulose comprises anionic nanofibrillar cellulose having an average fibril diameter of 200 nm or less, and to a method for preparing thereof. The present application also relates to the medical hydrogel for use for inducing vascularization in wounds and/or for treating deep wounds of dermis and/or below tissue.

Fig. 2

## Description

### Technical field

The present application relates to medical hydrogels comprising nanofibrillar cellulose and to methods for preparing thereof. More particularly the present application relates to medical hydrogels comprising nanofibrillar cellulose for treating wounds, especially for inducing vascularization in the wounds. The present application also relates to the medical hydrogel for use for inducing vascularization in wounds and/or for treating deep wounds of dermis and/or below tissue.

### Background

Wounds, especially deep wounds, may be challenging to treat. Especially wounds that involve damage of dermis or even deeper tissues, may heal poorly and heavy scarring may occur. In many cases, the patients' body grows scar tissues very fast, so that the deep wounds are not completely healed. Instead, only the surface of the wound is healed, while the inner membranes are not completely formed yet. Deeper and in some cases also dirtier and larger wounds are more susceptible to bacterial infection. As a result, this will delay the entire healing process.

Dressings may be applied to wounds. However, the dressings may stick to the wound and may not properly facilitate healing of deep wounds. There is need to improve the treatment of wounds, especially deep wounds, and to find better materials and methods for such treatment. Especially it is desired to improve the healing of the deep tissues. The materials should be mouldable to deep wounds and removable in such way that the wound is not further damaged but the healing of the deep wound would be initiated and maintained.

### Summary

It was found out that healing of deep wounds, such as wounds involving dermis and/or deeper tissues, can be improved by applying specific type of nanofibrillar cellulose hydrogel into and/or onto the wound. The hydrogel could induce vascularization or pre-vascularization in the deep tissues which significantly speeded up the healing process in the wound. Also the result was better as there was less scarring compared to if conventional materials were used for treating similar wound.

The present application provides a method for preparing a medical hydrogel, the method comprising

- providing anionically modified pulp, or
- providing pulp, and modifying the pulp anionically,
- disintegrating the anionically modified pulp until nanofibrillar cellulose having an average fibril diameter of 200 nm or less is obtained,
- optionally homogenizing the disintegrated anionically modified pulp in one or more non-fibrillating homogenizing step, and
- forming the nanofibrillar cellulose into a medical hydrogel having a content of nanofibrillar cellulose in the range of 1-3.5% (w/w).

The present application also provides a medical hydrogel comprising nanofibrillar cellulose, wherein the content of nanofibrillar cellulose in the hydrogel is in the range of 1-3.5% (w/w), and the nanofibrillar cellulose comprises anionic nanofibrillar cellulose having an average fibril diameter of 200 nm or less.

The present application also provides a medical hydrogel comprising nanofibrillar cellulose for use for inducing vascularization in wounds. The present application also provides a medical hydrogel comprising nanofibrillar cellulose for use for treating deep wounds of dermis and/or below tissue, preferably for inducing pre-vascularization.

The present application provides a method for preparing a tailored wound dressing, the method comprising

- providing the medical hydrogel disclosed herein,
- obtaining a digital three-dimensional model of an object designed to fit to a wound, and
- preparing a three-dimensional object from the medical hydrogel according to the digital three-dimensional model by additive manufacturing to obtain a wound dressing tailored to fit the wound.

The present application also provides a wound dressing tailored to fit a wound, the wound dressing comprising the medical hydrogel disclosed herein tailored to fit to the wound. The wound dressing may be obtained with the additive manufacturing method disclosed herein.

The present application also discloses a kit containing the medical hydrogel packed in one or more sealed package(s), or in one or more application device(s), such as a syringe, an applicator, a pump or a tube.

The main embodiments are characterized in the independent claims. Various embodiments are disclosed in the dependent claims. The embodiments and examples recited in the claims and the specification are mutually freely combinable unless otherwise explicitly stated.

It was found out that a hydrogel comprising nanofibrillar cellulose and having certain physical properties at specific ranges, especially relatively low viscosity and consistency, was especially suitable for medical purposes, especially as a medical product, such as a mouldable product, which can be used as a dressing or the like. When applied to a wound, the hydrogel exhibited properties such as solidity, low stickiness, sufficient fracture toughness expressed as compression work, formability, and good removability or detachability. It is important that the material does not stick to the wound, especially to the deep tissues, so when removing the hydrogel it does not damage the tissues or break into the wound. Also during the use it is important that the hydrogel remains in the wound and maintains a proper contact with the tissues. These properties were obtained with the present materials. Further, the material especially promoted healing of a deep wound or other damage or injury of such tissues.

The hydrogels disclosed herein provide good water retention capacity and molecule diffusion property speed, which properties are desired in medical applications such as wound treatment.

The hydrogels described herein are useful in medical applications, wherein the materials are in contact with living tissue. It was discovered that nanofibrillar cellulose exhibits unusual properties when it is applied onto a damaged area, especially when the damage is at deep tissues such as dermis, adipose tissue or other deep tissue. The products containing nanofibrillar cellulose as described herein are highly biocompatible with the living tissue and provide several advantageous effects. Without binding to any specific theory, it is believed that a hydrogel comprising very hydrophilic nanofibrillar cellulose having a very high specific surface area, and thus high water retention ability, when applied against a tissue, provides favourable moist environment between the tissue or wound and the nanofibrillar cellulose. The high amount of free hydroxyl groups in the nanofibrillar cellulose forms hydrogen bonds between the nanofibrillar cellulose and water molecules and enables gel formation and the high water retention ability of the nanofibrillar cellulose. Because of the high amount of water in the nanofibrillar cellulose hydrogel, mainly water is supposed to be in contact with tissue, and which also enables migration of fluids and/or agents from the wound to the hydrogel, or from the hydrogel to the wound. The relatively low viscosity and consistency of the products disclosed herein could surprisingly enhance these effects and provide further new effects.

One specific effect discovered when using the hydrogels described herein was that the vascularization at the damaged area was induced. This is especially advantageous when proper healing of the damaged area in a deep wound is desired. Without proper vascularization the damaged area would scar heavily resulting in incomplete healing. More particularly, the anionic nanofibrillar cellulose gel activated fibroblast cells in the skin, which in turn induced the formation of blood vessels in the damaged area *i.e.* vascularization. This effect was higher compared to vascularization obtained with chemically unmodified nanofibrillar cellulose gel with otherwise similar properties.

When the hydrogels are used for covering wounds or other damages or injuries, also other effects are provided. The usability of the products is good as the product may be applied and removed easily without being damaged, for example crumbled. The products maintained their form. The hydrogel protects the wound from infection and provides moist environment for the wound to heal. The hydrogel will not attach to a damaged skin, tissue or wound in such irreversible way as conventional materials do, which are usually very difficult to remove without damaging the healed or healing area. The conditions between the product and the skin facilitate the healing of a damaged area.

The hydrogels may also be used for controllably and effectively delivering or providing agents, such as therapeutic agents, to a subject, such as a patient or a user, for example by transdermal route or by other route. The controlled release refers for example to obtaining a desired release rate and/or profile of an agent or agents over a time period, which may be affected by the selection of the gel, for example the percentage of the gel or the thickness of the gel, the concentration or form of the releasable agent(s), presence of any auxiliary agents, or other conditions, such as pH, temperature and the like having an effect to the release rate and/or activity of the releasable agents. The combined effect of the special conditions between the tissue and the hydrogel as explained in previous and the release properties provides efficient delivery of substances into living tissue. On the other hand, the hydrogel may also absorb substances from the wound or damaged area, such as wound exudate. This may also promote healing. The nanofibrillar cellulose hydrogel provides a hydrophilic matrix, which is non-toxic, biocompatible and also biodegradable. For example the matrix may be degraded enzymatically. On the other hand the hydrogel is stable at physiological conditions.

**Brief description of the figures**

Figure 1 shows histopathological evaluation of wounds in humanized mice treated with NFC dressing (Type 1 dressing). A) The NOT treated injury; B) Type 1 dressing treated injury.

Figure 2 shows wound dressing studies with 3.2% anionic nanofibrillar cellulose (ANFC) hydrogel, unmodified nanofibrillar cellulose and control sample (no treatment). 2A shows photos of wounds surrounded by silicone rings sutured onto the surface of the skin surrounding the wound to prevent contraction, and wherein wound dressings are applied into the rings (except the controls). 2B shows a graph comparing the percentage of wound closure in 3 days and in 7 days.

Figure 3 shows results from the stimulation of the fibroblast activation. 3A shows $\alpha$-SMA staining to detect vascular networks and activation of fibroblasts in histological skin samples. 3B shows a graph presenting $\alpha$-SMA staining intensity in different samples.

Figure 4 shows chicken tests of 3.27% NFC hydrogels. 4A shows the hydrogel in the wound, and 4B shows the hydrogel removed from the wound.

Figure 5 shows chicken tests of 2.65% NFC hydrogels. 5A shows the hydrogel in the wound, and 5B shows the hydrogel removed from the wound.

**Detailed description**

In this specification, percentage values, unless specifically indicated otherwise, are based on weight (w/w). If any numerical ranges are provided, the ranges include also the upper and lower values. The open term "comprise" also includes a closed term "consisting of" as one option.

The materials and products described herein may be medical and/or scientific materials and products, such as life science materials and products, and may be used in methods and applications involving living tissue and/or bioactive material or substances, such as described herein. The materials or products may be or relate to materials or products for medical treatment, and may be used in methods wherein wounds, disorders and other damages of tissue, such as skin or tissue(s) below skin, is involved, and/or can be used for medical or scientific purposes, or in other related and applicable methods. Especially the present material and products are provided for applying to wounds, especially to deep wounds, such as wounds involving dermis and/or deeper tissue(s), *i.e.* wounds comprising a damage in said tissue(s), especially wherein treatment thereof is desired. Not all medical products, even in hydrogel form, are applicable to deep wounds, which require products having properties which do not damage or irritate the wound, especially the deep tissues, and which products can be at the same time moulded to fit into the wound and which products are also removable from the wound, preferably without damaging the wound and without breaking.

The term "medical" refers to a product or use wherein the product, *i.e.* a product comprising the hydrogel of the embodiments, is used or wherein the product is suitable for medical purposes. A medical product may be sterilized, or it is sterilisable, for example by using temperature, pressure, moisture, chemicals, radiation or a combination thereof, *i.e.* the product tolerates the sterilization treatment. The product may be for example autoclaved, or other methods using high temperature may be used. In one example the product is autoclaved at 121°C for 15 minutes. It may be also desired that a medical product is pyrogen free and it does not contain undesired protein residues, undesired bioactive material, animal-based material and/or the like. Also UV or gamma radiation sterilization may be used, especially for anionic nanofibrillar cellulose.

The nanofibrillar cellulose (NFC) hydrogel, such as anionic NFC hydrogel, is also able to sustainably or controllably release and/or retain active agents, such as therapeutic agents, for example pharmaceutical ingredients, or other bioactive agents as a function of time, especially when the temperature and pH are constant. One or more antibacterial or antimicrobial agent(s) and/or other therapeutic agent(s), which may facilitate the healing of the wound, may be included in the hydrogel. The content of such agent(s) in the hydrogel may be in the range of 0.01-1% (w/w), such as 0.05-0.5% (w/w), or another suitable physiologically effective amount.

The nanofibrillar cellulose may be provided at a suitable fibrillation degree, which may be characterized for example by the rheological properties of the material, and/or the diameter of fibrils or fibril bundles. For example the nanofibrillar cellulose, when dispersed in water, may provide a zero shear viscosity in the range of 1000-100000 Pa·s, for anionic grade preferably in the range of 1000-50000 Pa·s, such as 2000-20 000 Pa·s, and a yield stress in the range of 1-50 Pa, preferably 1-30 Pa, such as in the range of 3-15 Pa, determined by rotational rheometer at a consistency of 0.5% (w/w) by weight in aqueous medium at 22°C±1°C.

It was found out that the hydrogel for treating deep wounds and enhancing vascularization in the wounds should be preferably provided at a specific viscosity determined at the concentration of the hydrogel. The preferred viscosity was remarkably lower than in related prior art products and surprisingly such hydrogel could activate fibroblasts in the deep tissues and cause efficient vascularization in the deep wound. The hydrogel with said viscosity at the used concentrations remained well in the deep wound without causing damage or irritation to the deep tissues, and it was also easy to recover from the wound. The hydrogel maintained its activity, such as the vascularization effect, for several days. After this treatment period the wound was in a such healed and stable condition that it could be further treated and/or covered with other products, such as medicaments and/or other NFC hydrogels or NFC-containing products, such as gauzes, or treated surgically.

One embodiment provides a medical hydrogel comprising nanofibrillar cellulose, wherein the hydrogel has a viscosity in the range of 500- 2200 Pa·s, such as 500-2000 Pa·s, 500-1800 Pa·s, 700-2000 Pa·s or 1000-2000 Pa·s, measured with a viscometer at the concentration of the hydrogel and at 37°C at a shear rate of 0.1 1/s. In one embodiment the hydrogel has a viscosity in the range of 700-1800 Pa·s, such as 1000-1800 Pa·s, measured with a viscometer at the concentration of the hydrogel and at 37°C at a shear rate of 0.1 1/s. In one specific example corresponding to certain advantageous tested hydrogels, the viscosity is in the range of 1500-2000 Pa·s, measured with a viscometer at the concentration of the hydrogel and at 37°C at a shear rate of 0.1 1/s. These viscosities are measured at the concentration of the hydrogel as provided, *i.e.* "at own concentration", which may be in the range of 1-3.5% (w/w), as described herein, or a narrower range. The viscosities at own concentration may be measured with any suitable viscometer. In the tests the viscosities were measured with HAAKE Viscotester iQ Rheometer (Thermo Fisher Scientific, Karlsruhe, Germany) equipped with a Peltier system for temperature control. The viscosity may be called shear viscosity.

Also to control the properties of the hydrogel, especially when used as wound dressing in deep wounds or in further manufacturing steps, it is necessary to provide the hydrogel at a certain consistency (dry content) of the nanofibrillar cellulose, such as disclosed herein. The hydrogel contains usually mainly or only aqueous liquid, which may be water, or aqueous buffered solution and/or saline solution. The hydrogel may be formed in a physiological salt solution and therefore it may contain salt, such as 0.8-1.0% (w/w) salt, for example wherein the salt comprises sodium chloride.

The present application provides a medical hydrogel comprising nanofibrillar cellulose, wherein the content of nanofibrillar cellulose in the hydrogel is in the range of 1-3.5% (w/w), and the nanofibrillar cellulose comprises anionic nanofibrillar cellulose having an average fibril diameter of 200 nm or less. The medical hydrogel may be mouldable, especially hand-mouldable, so it is easy to mould the material to fit practically to any kind of wound. The nanofibrillar cellulose may be anionic nanofibrillar cellulose only, so no other type of nanofibrillar cellulose is present. The nanofibrillar cellulose may also be the only fibrillar, fibrous, polymeric and/or organic substance in the medical hydrogel, so in such case there may be no other matrix and/or reinforcing material in addition to nanofibrillar cellulose. Preferably the medical hydrogel does not contain other reinforcing and/or matrix-like materials or structures, for example synthetic, semi-synthetic or natural polymers or other compounds, such as collagens, proteins or peptides or other biological polymers, or plastic polymers.

The useful consistency of the nanofibrillar cellulose is relative narrow, in the range of 1-3.5% (w/w). At this consistency the hydrogel contains a suitable amount of water, is well processable and has properties suitable for a variety of medical uses disclosed herein, especially for promoting vascularization in deep wounds. The content of nanofibrillar cellulose in the hydrogel may be in the range of 1.1-3.5% (w/w). Depending on the wound types and dimensions it may be desired to use hydrogel which does not crumble or otherwise break easily. In such cases the content of nanofibrillar cellulose in the hydrogel may be in the range of 1.1-3.3% (w/w), or 1.1-3.0% (w/w), such as 1.5-3.5% (w/w), 1.5-3.3% (w/w) or 2.0-3.5% (w/w). However, on the other hand the lower concentrations may produce gels which are sticky but may be flexible, and thus may remain better in a wound or target which is prone to move. It was found especially important when applying the hydrogel into a deep wound to use hydrogel having the mentioned consistencies or nanofibrillar cellulose contents to avoid damaging the sensitive deeper tissues and to facilitate the vascularization in the deep tissues. In the tests hydrogels having a consistency in the range of 2.0-3.5% (w/w) were found advantageous for most uses, including manual moulding and preparing preshaped products. Also the healing properties for most deep wounds were especially good at said range.

In some cases, for example for 3D printing applications and/or to obtain a more mouldable, tough, less sticky and/or viscous hydrogel, it may be desired to use higher consistencies of the nanofibrillar cellulose, such as 2.0-3.5% (w/w), for example 2.5-3.5% (w/w).

Certain advantageous properties of the hydrogel comprising nanofibrillar cellulose include flexibility, elasticity and remouldability. As the hydrogel contains a lot of water, it may also show good diffusion and release properties of molecules. These properties are useful for example when the hydrogel is used as a cover in healing wounds, or in other medical applications, such as for delivering, storing and controlling bioactive agents.

Flexibility is a feature which is desired in many applications, such as in medical applications. For example flexible patches and dressings comprising nanofibrillar cellulose hydrogel are useful for applying onto skin, for example for covering wounds and other damages or injuries, such as burns. A related desired feature is mouldability, especially when the hydrogel is to be applied to a deep wound. Viscosity in general correlates with the mouldability. The viscosity must be high enough so that the structure of the composition is maintained, but on the other hand low enough to enable mouldability and to prevent cracking of the gel. Further, with an optimal initial viscosity the viscosity of the pseudoplastic product decreases during moulding to facilitate the moulding of the gel into a desired form.

For treating deep wound and to enhance the vascularization on the deep wound a specific water retention capacity may be required. One embodiment provides a medical hydrogel wherein the nanofibrillar cellulose has a water retention value in the range of 20-70 g water/g dry hydrogel, preferably 23-50 g water/g dry hydrogel, for example 25-35 g water/g dry hydrogel, more specifically less than 30 g water/g dry hydrogel, such as in the range of 20-29 g water/g dry hydrogel or 25-29 g water/g dry hydrogel. The water retention value may be measured with ÅAGWR (Åbo Akademi Gravitometric Water Retention) method.

The present application presents a method for preparing a medical hydrogel, the method comprising

- providing anionically modified pulp, such as wood pulp,
- disintegrating the pulp until nanofibrillar cellulose having an average fibril diameter of 200 nm or less is obtained,
- optionally homogenizing the disintegrated pulp in one or more non-fibrillating homogenizing step, and
- forming the nanofibrillar cellulose into a medical hydrogel having a content of nanofibrillar cellulose in the range of 1-3.5% (w/w), such as 1.1-3.5% (w/w), for example 2.0-3.5% (w/w). The method may comprise providing pulp and modifying the pulp anionically, preferably to obtain a desired modification degree, such as anionic charge.

The method may comprise forming the nanofibrillar cellulose into a medical hydrogel having a viscosity in the range of 500-2200 Pa·s, such as in the range of 700-1800 Pa·s or other viscosity disclosed herein, measured with a viscometer at the concentration of the hydrogel and at 37°C at a shear rate of 0.1 1/s

The medical hydrogels described herein may be obtained with this method.

The medical hydrogel of may be provided for use for inducing vascularization in wounds. More particularly, the medical hydrogel may be provided for use for treating deep wounds of dermis and/or below tissue, preferably for or by inducing vascularization and/or for activating fibroblasts. The medical hydrogels may be provided as packed in a sealed packing or applicator, and optionally in a kit. The packing or the kit may comprise instructions to use the medical hydrogel to a specific purpose and/or in a specific method, such as the ones disclosed herein.

A healthy skin comprises three main components or layers, the epidermis, dermis and hypodermis. The epidermis contains mainly keratinocytes and is primarily responsible for the barrier function of the skin, including the prevention of harmful pathogens from entering the body. It includes, from the outer layer to the inner layer, stratum corneum, stratum lucidum, stratum granulosum, stratum spinosum, and the stratum basale. Underneath the stratum basale the connecting layer can be found between the epidermis and the dermis, known as the basement membrane zone. The dermis comprises fibroblasts and extracellular matrix components such as collagen and elastin, blood vessels, nerves, and appendageal structures. It contains two layers, the papillary dermis (the upper layer), which is responsible for the prevention of sliding between the epidermis and the dermis and the provision of oxygen and nutrients to the epidermis, and the reticular dermis (the lower layer), which is mainly responsible for the mechanical properties of the skin. The hypodermis, also called the subcutaneous tissue, contains mostly adipose tissue

*Nanofibrillar cellulose*

The starting material for forming the medical hydrogel is nanofibrillar cellulose, also called as nanocellulose, which refers to isolated cellulose fibrils or fibril bundles derived from cellulose raw material. Nanofibrillar cellulose is based on a natural polymer that is abundant in nature. Nanofibrillar cellulose has a capability of forming viscous hydrogel in water. Nanofibrillar cellulose production techniques may be based on disintegrating fibrous raw material, such as grinding of aqueous dispersion of pulp fibers to obtain nanofibrillated cellulose. After the grinding or homogenization process, the obtained nanofibrillar cellulose material is a dilute viscoelastic hydrogel.

The obtained material usually exists at a relatively low concentration homogeneously distributed in water due to the disintegration conditions. The starting material may be an aqueous gel at a concentration of 0.2-10% (w/w), for example 0.2-5% (w/w). The nanofibrillar cellulose may be obtained directly from the disintegration of fibrous raw material. An example of commercially available nanofibrillar cellulose hydrogel is Grow-Dex® by UPM.

Because of its nanoscale structure nanofibrillar cellulose has unique properties which enable functionalities which cannot be provided by conventional cellulose. However, because of the nanoscale structure nanofibrillar cellulose is also a challenging material. For example dewatering or handling of nanofibrillar cellulose may be difficult.

The nanofibrillar cellulose may be prepared from cellulose raw material of plant origin, or it may also be derived from certain bacterial fermentation processes. The nanofibrillar cellulose is preferably made of plant material. The raw material may be based on any plant material that contains cellulose. In one example the fibrils are obtained from non-parenchymal plant material. In such case the fibrils may be obtained from secondary cell walls. One abundant source of such cellulose fibrils is wood fibres. The nanofibrillar cellulose may be manufactured by homogenizing wood-derived fibrous raw material, which may be chemical pulp. Cellulose fibers are disintegrated to produce fibrils which have an average diameter of only some nanometers, which may be 200 nm or less in most cases, and gives a dispersion of fibrils in water. The fibrils originating from secondary cell walls are essentially crystalline with degree of crystallinity of at least 55%. Such fibrils may have different properties than fibrils originated from primary cell walls, for example the dewatering of fibrils originating from secondary cell walls may be more challenging. In general in the cellulose sources from primary cell walls, such as sugar beet, potato tuber and banana rachis, the microfibrils are easier to liberate from the fibre matrix than fibrils from wood, and the disintegration requires less energy. However, these materials are still somewhat heterogeneous and consist of large fibril bundles.

Non-wood material may be from agricultural residues, grasses or other plant substances such as straw, leaves, bark, seeds, hulls, flowers, vegetables or fruits from cotton, corn, wheat, oat, rye, barley, rice, flax, hemp, manila hemp, sisal hemp, jute, ramie, kenaf, bagasse, bamboo or reed. The cellulose raw material may be also derived from the cellulose-producing micro-organism. The micro-organisms may be of the genus *Acetobacter, Agrobacterium, Rhizobium, Pseudomonas or Alcaligenes,* preferably of the genus *Acetobacter* and more preferably of the species *Acetobacter xylinum or Acetobacter pasteurianus.*

It was found out that nanofibrillar cellulose obtained from wood cellulose is preferable for medical or scientific products described herein. Wood cellulose is available in large amounts, and the preparation methods developed for wood cellulose enable producing nanofibrillar materials suitable for the products. The nanofibrillar cellulose obtained by fibrillating plant fibers, especially wood fibers, differs structurally from nanofibrillar cellulose obtained from microbes, and it has different properties. For example compared to bacterial cellulose, nanofibrillated wood cellulose is homogenous and more porous and loose material, which is advantageous in applications involving living cells. Bacterial cellulose is usually used as such without similar fibrillation as in plant cellulose, so the material is different also in this respect. Bacterial cellulose is dense material which easily forms small spheroids and therefore the structure of the material is discontinuous, and it may be not desired to use such material in the applications relating to living cells, especially when homogeneity of the material is required. In one example the cellulose is non-bacterial cellulose.

Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. In one example the nanofibrillar cellulose is obtained from wood pulp. The nanofibrillar cellulose may be obtained from hardwood pulp. In one example the hardwood is birch. The nanofibrillar cellulose may be obtained from softwood pulp. In one example said wood pulp is chemical pulp. Chemical pulp may be desired for the products disclosed herein. Chemical pulp is pure material and may be used in a wide variety of applications. For example chemical pulp lack the pitch and resin acids present in mechanical pulp, and it is more sterile or easily sterilisable. Further, chemical pulp is more flexible and provides advantageous properties for example in medical and scientific materials. For example very homogenous nanofibrillar cellulose materials may be prepared without excess processing or need for specific equipment or laborious process steps.

Nanofibrillar cellulose, including the cellulose fibrils and/or fibril bundles, is characterized by a high aspect ratio (length/diameter). The average length of nanofibrillar cellulose (the median length of particles such as fibrils or fibril bundles) may exceed 1 $\mu$m, and in most cases it is 50 $\mu$m or less. If the elementary fibrils are not completely separated from each other, the entangled fibrils may have an average total length for example in the range of 1-100 $\mu$m, 1-50 $\mu$m, or 1-20 $\mu$m. However, if the nanofibrillar material is highly fibrillated, the elementary fibrils may be completely or almost completely separated and the average fibril length is shorter, such as in the range of 1-10 $\mu$m or 1-5 $\mu$m. This applies especially for native grades of fibrils which are not shortened or digested, for example chemically, enzymatically or mechanically. However, strongly derivatized nanofibrillar cellulose may have a shorter average fibril length, such as in the range of 0.3-50 $\mu$m, such as 0.3-20 $\mu$m, for example 0.5-10 $\mu$m or 1-10 $\mu$m. Especially shortened fibrils, such as enzymatically or chemically digested fibrils, or mechanically treated material, may have an average fibril length of less than 1 $\mu$m, such as 0.1-1 $\mu$m, 0.2-0.8 $\mu$m or 0.4-0.6 $\mu$m. The fibril length and/or diameter may be estimated microscopically, for example using CRYO-TEM, SEM or AFM images.

The average diameter (width) of nanofibrillar cellulose is less than 1 $\mu$m, or 500 nm or less, such as in the range of 1-500 nm, but preferably 200 nm or less, even 100 nm or less or 50 nm or less, such as in the range of 1-200 nm, 2-200 nm, 2-100 nm, or 2-50 nm, even 2-20 for highly fibrillated material. The diameters disclosed herein may refer to fibrils and/or fibril bundles. The smallest fibrils are in the scale of elementary fibrils, the average diameter being typically in the range of 2-12 nm. The dimensions and size distribution of the fibrils depend on the refining method and efficiency. In case of highly refined native nanofibrillar cellulose, the average fibril diameter, including fibril bundles, may be in the range of 2-200 nm or 5-100 nm, for example in the range of 10-50 nm. Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose obtained from plants, especially wood, may also contain small amounts of other plant components, especially wood components, such as hemicellulose or lignin. The amount is dependent on the plant source.

In general cellulose nanomaterials may be divided into categories according to TAPPI W13021, which provides standard terms for cellulose nanomaterials. Not all of these materials are nanofibrillar cellulose. Two main categories are "Nano objects" and "Nano structured materials". Nanostructured materials include "Cellulose microcrystals" (sometimes called as CMC) having a diameter of 10-12 $\mu$m and length:diameter ratio (L/D) <2, and "Cellulose microfibrils" having a diameter of 10-100 nm and a length of 0.5-50 $\mu$m. Nano objects include "Cellulose nanofibers", which can be divided into "Cellulose nanocrystals" (CNC) having a diameter of 3-10 nm and L/D >5, and "Cellulose nanofibrils" (CNF or NFC), having a diameter of 5-30 nm and L/D >50. It may be defined that the nanofibrillar cellulose disclosed herein is not or does not contain cellulose microcrystals or cellulose nanocrystals.

Different grades of nanofibrillar cellulose may be categorized based on three main properties: (i) size distribution, length and diameter (ii) chemical composition, and (iii) rheological properties. To fully describe a grade, the properties may be used in parallel. Examples of different grades include native (or chemically unmodified) NFC, oxidized NFC (high viscosity), oxidized NFC (low viscosity), carboxymethylated NFC and cationized NFC. Within these main grades, also sub-grades exist, for example: extremely well fibrillated vs. moderately fibrillated, high degree of substitution vs. low degree of substitution, low viscosity vs. high viscosity etc. The fibrillation technique and the chemical pre-modification have an influence on the fibril size distribution. Typically, non-ionic grades have wider average fibril diameter (for example in the range of 10-100 nm, or 10-50 nm) while the chemically modified grades are a lot thinner (for example in the range of 2-20 nm). Distribution is also narrower for the modified grades. Certain modifications, especially TEMPO-oxidation, yield shorter fibrils.

Depending on the raw material source, e.g. hardwood vs. softwood pulp, different polysaccharide composition exists in the final nanofibrillar cellulose product. Commonly, the non-ionic grades are prepared from bleached birch pulp, which yields high xylene content (25% by weight). Modified grades are prepared either from hardwood or softwood pulps. In those modified grades, the hemicelluloses are also modified together with the cellulose domain. Most probably, the modification is not homogeneous, i.e. some parts are more modified than others. Thus, detailed chemical analysis is usually not possible as the modified products are complicated mixtures of different polysaccharide structures.

In an aqueous environment, a dispersion of cellulose nanofibers forms a viscoelastic hydrogel network. The gel is formed already at relatively low concentrations of for example 0.05-0.2% (w/w) by dispersed and hydrated entangled fibrils. The viscoelasticity of the NFC hydrogel may be characterized for example with dynamic oscillatory rheological measurements.

The nanofibrillar cellulose hydrogels exhibit characteristic rheological properties. For example they are shear-thinning or pseudoplastic materials, which may be considered as a special case of thixotropic behavior, which means that their viscosity depends on the speed (or force) by which the material is deformed. When measuring the viscosity in a rotational rheometer, the shear-thinning behavior is seen as a decrease in viscosity with increasing shear rate. The hydrogels show plastic behavior, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity is seen after exceeding the critical shear stress. The zero shear viscosity and the yield stress are the most important rheological parameters to describe the suspending power of the materials. These two parameters separate the different grades quite clearly and thus enable classification of the grades.

The dimensions of the fibrils or fibril bundles are dependent for example on the raw material, the disintegration method and number of disintegration runs. Mechanical disintegration of the cellulose raw material may be carried out with any suitable equipment such as a refiner, grinder, disperser, homogenizer, colloider, friction grinder, pin mill, rotor-rotor dispergator, ultrasound sonicator, fluidizer such as microfluidizer, macrofluidizer or fluidizer-type homogenizer. The disintegration treatment is performed at conditions wherein water is sufficiently present to prevent the formation of bonds between the fibers.

In one example the disintegration is carried out by using a disperser having at least one rotor, blade or similar moving mechanical member, such as a rotor-rotor dispergator, which has at least two rotors. In a disperser the fiber material in dispersion is repeatedly impacted by blades or ribs of rotors striking it from opposite directions when the blades rotate at the rotating speed and at the peripheral speed determined by the radius (distance to the rotation axis) in opposite directions. Because the fiber material is transferred outwards in the radial direction, it crashes onto the wide surfaces of the blades, i.e. ribs, coming one after the other at a high peripheral speed from opposite directions; in other words, it receives a plurality of successive impacts from opposite directions. Also, at the edges of the wide surfaces of the blades, i.e. ribs, which edges form a blade gap with the opposite edge of the next rotor blade, shear forces occur, which contribute to the disintegration of the fibers and detachment of fibrils. The impact frequency is determined by the rotation speed of the rotors, the number of the rotors, the number of blades in each rotor, and the flow rate of the dispersion through the device.

In a rotor-rotor dispergator the fiber material is introduced through counter-rotating rotors, outwards in the radial direction with respect to the axis of rotation of the rotors in such a way that the material is repeatedly subjected to shear and impact forces by the effect of the different counter-rotating rotors, whereby it is simultaneously fibrillated. One example of a rotor-rotor dispergator is an Atrex device.

Another example of a device suitable for disintegrating is a pin mill, such as a multi-peripheral pin mill. One example of such device includes a housing and in it a first rotor equipped with collision surfaces; a second rotor concentric with the first rotor and equipped with collision surfaces, the second rotor being arranged to rotate in a direction opposite to the first rotor; or a stator concentric with the first rotor and equipped with collision surfaces. The device includes a feed orifice in the housing and opening to the center of the rotors or the rotor and stator, and a discharge orifice on the housing wall and opening to the periphery of the outermost rotor or stator.

In one example the disintegrating is carried out by using a homogenizer. In a homogenizer the fiber material is subjected to homogenization by an effect of pressure. The homogenization of the fiber material dispersion to nanofibrillar cellulose is caused by forced through-flow of the dispersion, which disintegrates the material to fibrils. The fiber material dispersion is passed at a given pressure through a narrow through-flow gap where an increase in the linear velocity of the dispersion causes shearing and impact forces on the dispersion, resulting in the removal of fibrils from the fiber material. The fiber fragments are disintegrated into fibrils in the fibrillating step.

As used herein, the term "fibrillation" generally refers to disintegrating fiber material mechanically by work applied to the particles, where cellulose fibrils are detached from the fibers or fiber fragments. The work may be based on various effects, like grinding, crushing or shearing, or a combination of these, or another corresponding action that reduces the particle size. The expressions "disintegration" or "disintegration treatment" may be used interchangeably with "fibrillation".

The fiber material dispersion that is subjected to fibrillation is a mixture of fiber material and water, also herein called "pulp". The fiber material dispersion may refer generally to whole fibers, parts (fragments) separated from them, fibril bundles, or fibrils mixed with water, and typically the aqueous fiber material dispersion is a mixture of such elements, in which the ratios between the components are dependent on the degree of processing or on the treatment stage, for example number of runs or "passes" through the treatment of the same batch of fiber material.

One way to characterize the nanofibrillar cellulose is to use the viscosity of an aqueous solution containing said nanofibrillar cellulose. The viscosity may be for example Brookfield viscosity or zero shear viscosity. The specific viscosity, as described herein, distinguishes nanofibrillar cellulose from non-nanofibrillar cellulose.

In one example the apparent viscosity of the nanofibrillar cellulose is measured with a Brookfield viscometer (Brookfield viscosity) or another corresponding apparatus. Suitably a vane spindle (number 73) is used. There are several commercial Brookfield viscometers available for measuring apparent viscosity, which all are based on the same principle. Suitably RVDV spring (Brookfield RVDV-III) is used in the apparatus. A sample of the nanofibrillar cellulose is diluted to a concentration of 0.8% by weight in water and mixed for 10 min. The diluted sample mass is added to a 250 ml beaker and the temperature is adjusted to $20°C \pm 1$ °C, heated if necessary and mixed. A low rotational speed 10 rpm is used. In general Brookfield viscosity may be measured at $20°C \pm 1$ °C, at a consistency of 0.8% (w/w) and at 10 rpm.

The nanofibrillar cellulose, for example provided as a starting material in the method, may be characterized by the viscosity it provides in a water solution. The viscosity describes, for example, the fibrillation degree of the nanofibrillar cellulose. In one example the nanofibrillar cellulose when dispersed in water provides a Brookfield viscosity of at least 2000 mPa·s, such as at least 3000 mPa·s, measured at $20°C \pm 1$ °C, at a consistency of 0.8% (w/w) and at 10 rpm. In one example the nanofibrillar cellulose, when dispersed in water, provides a Brookfield viscosity of at least 10000 mPa·s measured at $20°C \pm 1°C$, at a consistency of 0.8% (w/w) and at 10 rpm. In one example the nanofibrillar cellulose, when dispersed in water, provides a Brookfield viscosity of at least 15000 mPa·s measured at $20°C \pm 1°C$, at a consistency of 0.8% (w/w) and at 10 rpm. Examples of Brookfield viscosity ranges of said nanofibrillar cellulose when dispersed in water include 2000-20000 mPa·s, 3000-20000 mPa·s, 10000-20000 mPa·s, 15000-20000 mPa·s, 2000-25000 mPa·s, 3000-25000 mPa·s, 10000-25000 mPa·s, 15000-25000 mPa·s, 2000-30000 mPa·s, 3000-30000 mPa·s, 10000-30000 mPa·s, and 15000-30000 mPa·s, measured at $20°C \pm 1°C$, at a consistency of 0.8% (w/w) and at 10 rpm.

The nanofibrillar cellulose may also be characterized by the average diameter (or width), or by the average diameter together with the viscosity, such as Brookfield viscosity or zero shear viscosity, and also with yield stress or storage modulus. In one example nanofibrillar cellulose suitable for use in the products described herein has an average fibril diameter in the range of 1-200 nm, or 1-100 nm. In one example said nanofibrillar cellulose has an average fibril diameter in the range of 1-50 nm, such as 2-20 nm or 5-30 nm. In one example said nanofibrillar cellulose has an average fibril diameter in the range of 2-15 nm, such as in the case of (TEMPO) oxidized nanofibrillar cellulose.

The diameter of a fibril may be determined with several techniques, such as by microscopy. Fibril thickness and width distribution may be measured by image analysis of the images from a field emission scanning electron microscope (FE-SEM), a transmission electron microscope (TEM), such as a cryogenic transmission electron microscope (cryo-TEM), or an atomic force microscope (AFM). In general AFM and TEM suit best for nanofibrillar cellulose grades with narrow fibril diameter distribution.

A rheometer viscosity of the nanofibrillar cellulose dispersion may be measured at 22°C with a stress controlled rotational rheometer (AR-G2, TA Instruments, UK) equipped with a narrow gap vane geometry (diameter 28 mm, length 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s$^{-1}$ is exceeded. This method may be used for determining the zero-shear viscosity.

In one example the nanofibrillar cellulose, for example provided as a starting material in the method, when dispersed in water, provides a zero shear viscosity ("plateau" of constant viscosity at small shearing stresses) in the range of 1000-50000 Pa·s, such as in the range of 2000-20000 Pa·s, and a yield stress (shear stress where the shear thinning begins) in the range of 1-30 Pa, such as in the range of 3-15 Pa, determined by rotational rheometer at a consistency of 0.5% (w/w) by weight in aqueous medium at 22°C±1°C. In one example the nanofibrillar cellulose provides a zero shear viscosity in the range of 5000-50000 Pa·s and a yield stress in the range of 3-15 Pa. Such nanofibrillar cellulose may also have an average fibril diameter of 200 nm or less, such as in the range of 1-200 nm.

In one embodiment the nanofibrillar cellulose has an average diameter of a fibril in the range of 1-200 nm and/or, when dispersed in water, provides a storage modulus of 350 Pa or more, such as in the range of 350-5000 Pa, or preferably 350-1000 Pa, and yield stress of 25 Pa or more, such as in the range of 25-300 Pa, preferably 25-75 Pa, determined by stress controlled rotational rheometer with gradually increasing shear stress in a range of 0.001-100 Pa at a frequency 10 rad/s, strain 2%, at 25°C.

Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry is used. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1 %. HACH P2100 Turbidometer with a 50 ml measuring vessel is used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

One way to characterize nanofibrillar cellulose is to define both the viscosity and the turbidity. Low turbidity refers to small size of the fibrils, such as small diameter, as small fibrils scatter light poorly. In general as the fibrillation degree increases, the viscosity increases and at the same time the turbidity decreases. This happens, however, until a certain point. When the fibrillation is further continued, the fibrils finally begin to break and cannot form a strong network any more. Therefore, after this point, both the turbidity and the viscosity begin to decrease.

In one example the turbidity of anionic nanofibrillar cellulose is lower than 90 NTU, for example from 3 to 90 NTU, such as from 5 to 60, for example 8-40 measured at a consistency of 0.1% (w/w) in aqueous medium, and measured by nephelometry. In one example the turbidity of native nanofibrillar may be even over 200 NTU, for example from 10 to 220 NTU, such as from 20 to 200, for example 50-200 measured at measured at 20°C±1°C a consistency of 0.1% (w/w) in aqueous medium, and measured by nephelometry. To characterize the nanofibrillar cellulose these ranges may be combined with the viscosity ranges of the nanofibrillar cellulose, such as nanofibrillar cellulose which, when dispersed in water, provides a Brookfield viscosity of at least 2000 mPa·s, at least 3000 mPa·s, at least 5000 mPa·s, such as at least 10000 mPa·s, for example at least 15000 mPa·s measured at 20°C±1°C, at a consistency of 0.8% (w/w) and at 10 rpm.

Nanofibrillar cellulose may be or comprise non-modified nanofibrillar cellulose. The drainage of non-modified nanofibrillar cellulose is significantly faster than for example anionic grade. Non-modified nanofibrillar cellulose generally has a Brookfield viscosity in the range of 2000-10000 mPa·s, measured at 20°C±1°C, at a consistency of 0.8% (w/w) and at 10 rpm. It is preferred that the nanofibrillar cellulose has a suitable carboxylic acid content, such as in the range of 0.6-1.4 mmol COOH/g, for example in the range of 0.7-1.2 mmol COOH/g, or in the range of 0.7-1.0 mmol COOH/g or 0.8-1.2 mmol COOH/g, determined by conductometric titration.

The disintegrated fibrous cellulosic raw material may be modified fibrous raw material. Modified fibrous raw material means raw material where the fibers are affected by the treatment so that cellulose nanofibrils are more easily detachable from the fibers. The modification is usually performed to fibrous cellulosic raw material which exists as a suspension in a liquid, i.e. pulp.

The modification treatment to the fibers may be chemical, enzymatic or physical. In chemical modification the chemical structure of cellulose molecule is changed by chemical reaction ("derivatization" of cellulose), preferably so that the length of the cellulose molecule is not affected but functional groups are added to β-D-glucopyranose units of the polymer. The chemical modification of cellulose takes place at a certain conversion degree, which is dependent on the dosage of reactants and the reaction conditions, and as a rule it is not complete so that the cellulose will stay in solid form as fibrils and does not dissolve in water. In physical modification anionic, cationic, or non-ionic substances or any combination of these are physically adsorbed on cellulose surface.

The cellulose in the fibers may be especially ionically charged after the modification. The ionic charge of the cellulose weakens the internal bonds of the fibers and will later facilitate the disintegration to nanofibrillar cellulose. The ionic charge may be achieved by chemical or physical modification of the cellulose. The fibers may have higher anionic or cationic charge after the modification compared with the starting raw material. Most commonly used chemical modification methods for making an anionic charge are oxidation, where hydroxyl groups are oxidized to aldehydes and carboxyl groups, sulphonization and carboxymethylation. Chemical modifications introducing groups, such as carboxyl groups, which may take part in forming a covalent bond between the nanofibrillar cellulose and the bioactive molecule, may be desired. A cationic charge in turn may be created chemically by cationization by attaching a cationic group to the cellulose, such as quaternary ammonium group.

Nanofibrillar cellulose may comprise chemically modified nanofibrillar cellulose, which may generally include for example anionic or cationic modification. In one example the nanofibrillar cellulose is anionically modified nanofibrillar cellulose. In one example the nanofibrillar cellulose is not cationically modified nanofibrillar cellulose. In one example the anionically modified nanofibrillar cellulose is oxidized nanofibrillar cellulose. In one example the anionically modified nanofibrillar cellulose is sulphonized nanofibrillar cellulose. In one example the anionically modified nanofibrillar cellulose is carboxymethylated nanofibrillar cellulose. The material obtained with the anionical modification of cellulose may be called anionic cellulose, which refers to material wherein the amount or proportion of anionic groups, such as carboxylic groups, is increased by the modification, when compared to a non-modified material. It is also possible to introduce other anionic groups to the cellulose, instead or in addition to carboxylic groups, such as phosphate groups or sulphate groups. The content of these groups may be in the same ranges as is disclosed for carboxylic acid herein.

The cellulose may be oxidized. In the oxidation of cellulose, the primary hydroxyl groups of cellulose may be oxidized catalytically by a heterocyclic nitroxyl compound, such as through N-oxyl mediated catalytic oxidation, for example 2,2,6,6-tetramethylpiperidinyl-1-oxy free radical, generally called "TEMPO". The primary hydroxyl groups (C6-hydroxyl groups) of the cellulosic β-D-glucopyranose units are selectively oxidized to carboxylic groups. Some aldehyde groups are also formed from the primary hydroxyl groups. Regarding the finding that low degree of oxidation does not allow efficient enough fibrillation and higher degree of oxidation inflicts degradation of cellulose after mechanical disruptive treatment, the cellulose may be oxidized to a level having a carboxylic acid content in the oxidized cellulose in the range of 0.5-2.0 mmol COOH/g pulp, 0.6-1.4 mmol COOH/ g pulp, or 0.8-1.2 mmol COOH / g pulp, preferably to 1.0-1.2 mmol COOH/ g pulp, determined by conductometric titration. When the fibers of oxidized cellulose so obtained are disintegrated in water, they give stable transparent dispersion of individualized cellulose fibrils, which may be, for example, of 3-5 nm in width. With oxidized pulp as the starting medium, it is possible to obtain highly fibrillated nanofibrillar cellulose where Brookfield viscosity measured at a consistency of 0.8% (w/w) is at least 10000 mPa·s, for example in the range of 10000-30000 mPa·s.

Whenever the catalyst "TEMPO" is mentioned in this disclosure, it is evident that all measures and operations where "TEMPO" is involved apply equally and analogously to any derivative of TEMPO or any heterocyclic nitroxyl radical capable of catalyzing selectively the oxidation of the hydroxyl groups of C6 carbon in cellulose.

The modifications of nanofibrillar cellulose disclosed herein may also be applied to other fibrillar cellulose grades described herein. For example also highly refined cellulose or microfibrillar cellulose may be similarly chemically or enzymatically modified. However, there are differences for example in the final fibrillation degree of the materials.

In one example such chemically modified nanofibrillar cellulose, when dispersed in water, provides a Brookfield viscosity of at least 10000 mPa·s, at least 15000 mPa·s, or at least 18000 mPa·s measured at 20°C±1°C, at a consistency of 0.8% (w/w) and at 10 rpm. Examples of anionic nanofibrillar celluloses used have a Brookfield viscosity in the range of 13000-25000 mPa·s, 13000-20000 mPa·s, or 15000-20000 mPa·s, depending on the degree of fibrillation.

In one example the nanofibrillar cellulose is TEMPO oxidized nanofibrillar cellulose. It provides high viscosity at low concentrations, for example a Brookfield viscosity of at least 20000 mPa·s, even at least 25000 mPa·s, measured at 20°C±1°C, at a consistency of 0.8% (w/w) and at 10 rpm. In one example the Brookfield viscosity of TEMPO oxidized nanofibrillar cellulose is in the range of 20000-30000 mPa·s, such as 25000-30000 mPa·s, measured at 20°C±1°C, at a consistency of 0.8% (w/w) and at 10 rpm.

In general chemically unmodified nanofibrillar cellulose, when dispersed in water, provides a lower Brookfield viscosity of at least 2000 mPa·s, or at least 3000 mPa·s, measured at 20°C±1°C, at a consistency of 0.8% (w/w) and at 10 rpm.

Auxiliary agents for enhancing the manufacturing process or improving or adjusting the properties of the product may be included in the nanofibrillar cellulose dispersion. Such auxiliary agents may be soluble in the liquid phase of the dispersion, they may form an emulsion or they may be solid. Auxiliary agents may be added already during the manufacturing of the nanofibrillar cellulose dispersion to the raw material or they may be added to a formed nanofibrillar cellulose dispersion or gel. The auxiliary agents may be also added to the final product, for example by impregnating, spraying, dipping, soaking or the like method. The auxiliary agents are usually not covalently bound to the nanofibrillar cellulose, so they may be releasable from the nanocellulose matrix. A controlled and/or sustained release of such agents may be obtained when using NFC as matrix. Examples of auxiliary agents include therapeutic (pharmaceutic) agents and other agents affecting to the properties of the product or to the properties of the active agents, such as buffers, surfactants, plasticizers, emulsifiers or the like. In one example the dispersion contains one or more salts, which may be added to enhance the properties of the final product or to facilitate water removal from the product in the manufacturing process. Examples of salts include chloride salts, such as sodium chloride, calcium chloride and potassium chloride. The salt may be included in an amount in the range of 0.01-1.0% (w/w) of the dry matter in the dispersion. The final product may also be dipped or soaked in a solution of sodium chloride, such as in an aqueous solution of about 0.9% sodium chloride. Desired salt content in the final product may be in the range of 0.5-1%, such as about 0.9%, of the weight of the wet product. The salts, buffers and the like agents may be provided to obtain physiological conditions.

Multivalent cations may be included to obtain non-covalent crosslinking of the nanofibrillar cellulose. One example provides a nanofibrillar cellulose product comprising nanofibrillar cellulose, especially comprising anionically modified nanofibrillar cellulose, and multivalent cations, such as multivalent metal cations, for example selected from cations of calcium, magnesium, zinc, aluminum, gold, platinum and titanium, wherein the nanofibrillar cellulose is crosslinked by the multivalent cations. The amount of the multivalent cations may be in the range of 0.1-3% (w/w), for example 0.1-2% (w/w) calculated from the dry content of the hydrogel.

One example provides a method for preparing such a hydrogel, the method comprising providing pulp, disintegrating the pulp until nanofibrillar cellulose is obtained, and forming the obtained nanofibrillar cellulose into a hydrogel

The nanofibrillar cellulose may be fibrillated into a desired fibrillation degree and adjusted into desired water content, or otherwise modified, so that it forms a gel having desired properties as described herein. In one example the nanofibrillar cellulose in the hydrogel is anionically modified nanofibrillar cellulose.

The hydrogel to be used as a medical or scientific hydrogel needs to be homogenous. Therefore the method for preparing the hydrogel may include homogenizing a hydrogel comprising nanofibrillar cellulose, preferably with a homogenizing device such as ones described herein. With this preferably non-fibrillating homogenizing step it is possible to remove areas of discontinuity from the gel. A homogenous gel having better properties for the applications is obtained. The hydrogel may be further sterilized, for example by using heat and/or radiation, and/or by adding sterilizing agents, such as antimicrobials.

*Manufacture of objects*

The hydrogel disclosed herein may be also tailored to fit to a specific wound. The mouldability or formability of the hydrogel, especially a hydrogel having a content of nanofibrillar cellulose in the range of 2-3.5% (w/w), enables preparing such preformed or preshaped structures or products, which may be tailored according to specific need. One or more manufacturing device(s), mould(s) and or other devices or materials, such as computerized controlling devices for controlling the manufacturing device(s) and/or the process may be provided. Providing a preformed or preshaped product may be useful when treating deep wounds wherein it is not desired to mould the hydrogel in the wound to avoid damaging or irritating the deep tissues.

The preformed structures, which may be wound dressings, may be manufactured for example by moulding or by using additive manufacturing, generally known as 3D printing. Additive manufacturing is a process wherein material is joined or solidified under computer control to create a three-dimensional object. This can be carried out without a mold. The material may be added together layer by layer, for example by using and controlling one or more nozzles for providing or outputting the material.

Such method may require obtaining information about the three-dimensional structure of the wound or other damage whereto the tailored structure is to be applied. The information is used to prepare a digital three-dimensional model of the wound and/or of an object designed to fit to the wound. Therefore a digital three-dimensional model of an object designed to fit to the wound is obtained, and may be provided. This may be carried out by first scanning the target, such as wound or other damage, for example with a 3D scanner or other means for obtaining three-dimensional information of a target, and by using the three-dimensional information of the target creating a digital three-dimensional model of the target. The digital three-dimensional model may be configured to produce a desired object, such as the tailored wound dressing, when outputted with an additional manufacturing device. Therefore in one example the digital three-dimensional model is obtained from a 3D scan of the wound. The three-dimensional model of the wound may include the shape of the whole wound or a part thereof, or correspond to the shape.

Therefore provided is a method for preparing a tailored wound dressing, the method comprising

- providing the medical hydrogel,
- obtaining a digital three-dimensional model of an object designed to fit to a wound, and
- preparing a three-dimensional object from the medical hydrogel according to the digital three-dimensional model by additive manufacturing to obtain a wound dressing tailored to fit the wound.

Obtaining the information about the three-dimensional structure of the wound or other damage may be carried out by using a suitable imaging device or a system including such an imaging device capable of creating a three dimensional image or model of a target, also called as 3D imaging device. The imaging device may be a 3D scanning device. 3D scanning is the process of analyzing a real-world object or environment to collect data on its shape and possibly its appearance, such as colour. The collected data can then be used to construct digital 3D models. A 3D scanning devices may be classified in contact and non-contact types. A contact-type may not be desired in the case of wounds, so the imaging devices used herein are preferably non-contact types, which may be passive or active. Active scanners emit sound, radiation or light and detect its reflection or radiation passing through the object in order to probe an object or environment. Possible types of emissions used include light, ultrasound or x-ray. An active imaging device may be for example a 3D laser scanner, such as time-of-flight, triangulation, or conoscopic laser scanner; or a structured-light or a modulated light 3D scanner. A passive imaging device may be for example stereoscopic system, such as one including two cameras, or a photometric system.

The method may comprise providing a 3D imaging device, and obtaining a digital three-dimensional model of an object designed to fit to a wound by using the 3D imaging device.

The method may comprise providing an additive manufacturing device, such as a 3D printer or other suitable device comprising or configured to receive one or more container(s) for hydrogel, one or more nozzles or the like outputting means, one or more actuator(s) for moving the one or more nozzle(s), and one or more control unit(s) for controlling the movement of the one or more nozzle(s) to obtain a desired object by additive manufacturing. The final object may contain a shape corresponding to the shape of the scanned object, such as the wound, either fully or partially.

It is also possible to manufacture a wound dressing with a predetermined shape, such as in a general shape arranged to fit into one general shape of a wound. A series of wound dressing with different predetermined shapes, preferably arrange to fit into common shapes or types of wounds, may be manufactured and/or provided. Digital three-dimensional model(s) may or may not be used, and in one example the three-dimensional model(s) is not obtained by imaging an existing wound or a specific wound as discussed in previous. Instead the three-dimensional model may be created as a generic model, for example by designing with a computer. Also a manufacturing device comprising one or more mould(s) for moulding the wound dressing(s) may be provided and/or used for preparing such generic products. A series of generic wound dressings with different shapes may be provided, preferably packed in sealed packing(s), for example in a kit, which may further contain other wound dressing and/or healing product(s).

The nanofibrillar cellulose may be provided to a manufacturing device, such as a 3D printer, at the consistency desired in the final wound dressing. The nanofibrillar cellulose may be present as a dispersion or hydrogel having a concentration of 1.5-3.5% (w/w), 2.0-3.5% (w/w) or 2.5-3.5% (w/w), or the concentration may be lower or higher, and it is provided into a container or in a container, for example a syringe, an ampule, a bottle, a tube, a cassette or the like, which may be designed to be attached to the manufacturing device.

When 3D printing nanofibrillar cellulose, the process differs from the convention 3D printing processes. The cellulose does not melt when heated, so the methods and devices used for printing materials such as plastic, metal or the like may not be applicable. A specific manufacturing device may have to be used, such as a syringe-based device, which may utilize one or more syringes. Such a device may handle and print liquid, paste, gel or slurry, so it is suitable for nanofibrillar cellulose hydrogel materials. The manufacturing device may contain software operatively stored in a control unit configured to control the device, or the device may be connected to an external control unit, such as a computer, containing the software. The software, and therefore also the manufacturing device, may be configured to use the digital three-dimensional model as a template for manufacturing the three-dimensional object.

Nanofibrillar cellulose as shear-thinning or thixotropic material behaves in different way than conventional materials used in additive manufacturing. When shear forces are applied to the material, for example when the material is mixed, pressed and/or forced through a nozzle, the nanofibrillar cellulose gel exhibits thinning behavior which facilitates the flow of the material to the target. However, when the material has been printed or otherwise outputted from the manufacturing device, the viscosity will return back to the previous state of higher viscosity. This may provide challenges when forming the desired medical hydrogel structures to a support, but it may also facilitate the 3D printing process and formability of the desired structure. For example the shear thinning or thixotropy of the hydrogel may provide a suitable open time for the material wherein it can be molded and desired structures may be formed. There is usually no need to control the temperature, for example the material does not have to be heated and/or cooled for molding or setting purposes, but the process may be carried out at room or ambient temperatures.

The medical hydrogel structures may be manufactured, or printed, onto a support, which may be metal, plastic or other suitable material, or which may be coated with a non-stick coating, membrane or the like.

It may be necessary to dry, settle or fix the outputted material, so the method may contain a drying, settling or fixing step. For example vacuum or under pressure, and/or freezing and/or heating may be utilized for forming the structure and/or dewatering it. The drying, settling or fixing may be carried out for the final structure only or it may be repeated two or more times. When additive manufacturing is carried out by providing more than one layer, it may be necessary to let the material to dry, settle or fix 10-30 minutes, for example about 15 minutes, before applying a new layer. If the layers are applied manually, for example by using a syringe and injection needle, it may be necessary to let the layer dry, settle or fix for a longer time, such as 30-60 minutes before applying a new layer.

The outputting means, such as nozzle or the like, of the manufacturing device, may also play an important role. One example of the outputting device is a syringe, and it was found out that preferably a new and unused syringe should be used every time. A needle, preferably a metallic needle, may act as the nozzle. The needle may be an injection needle or the like, for example about 16 gauge needle, which has an nominal inner diameter of 1.194 mm. In general the nozzle may be an inner diameter in the range of 0.3-3.0 mm, such as 0.3-2.0 mm, for example 0.3-1.5 mm, 0.4-1.5 mm or 0.4-1.3 mm.

After the additive manufacturing it may be necessary to let the material, *i.e.* the medical hydrogel, to dry, settle or fix, for example for at least 30 minutes or at least 60 minutes. The medical hydrogels may be stored for at least two weeks after manufacture, especially when stored in refrigerator temperature, such as less than 10°C, for example 2-10°C or 4-8°C. The medical hydrogel may be packed in a sealed packing.

The present application provides a wound dressing tailored to fit a wound, the wound dressing comprising the medical hydrogel tailored to fit to the wound. Such product may be obtained with the additive manufacturing method disclosed in previous. Such tailored wound dressing is mechanically stable and retains its shape. As it is tailored to fit to the wound, applying the dressing to the wound does not cause unnecessary pain to the patient or damage the wound. In such case it may not be necessary to mould the dressing manually.

The wound dressing tailored to fit to a wound may contain only or mostly the nanofibrillar cellulose disclosed herein. However, it may be possible, for example in the case or large and/or extraordinary shaped wounds to include a supporting part to the dressing, for example an inner part or another layer coated with the nanofibrillar cellulose hydrogel. The supporting part is not necessary a part of the actual hydrogel and it may comprise different material(s) than the hydrogel, for example reinforcing material, such as non-nanofibrillar cellulose, plastic, or composite thereof.

The present application provides a kit containing the medical hydrogel packed in one or more sealed package(s), or in one or more application device(s), such as a syringe, an applicator, a pump or a tube. The kit may comprise instructions to use the medical hydrogel for inducing vascularization in wounds, and/or for treating deep wounds of dermis and/or below tissue, preferably for inducing vascularization, or for other purpose disclosed herein.

The kit may further comprise one or more sheet(s) of medical product comprising a gauze and nanofibrillar cellulose, preferably having a moisture content below 10% (w/w), such as in the range of 1-10% (w/w) or 5-10% (w/w). Such products may be used in a treatment method comprising first covering the wound with medical hydrogel disclosed herein, incubating, treating or waiting for a period of time, and replacing the medical hydrogel with a different medical product comprising a gauze and nanofibrillar cellulose. In such case the vascularization in the wound can be initiated, and after suitable time the conditions in the wound are different and a different medical product may be applied to the wound, which on its turn better facilitates the healing of the already vascularized wound. The first medical product, namely the hydrogel, may absorb substances from the wound at the first stage, for example harmful substances such as microbes, impurities, metabolites and the like, which may facilitate the healing process and make the wound susceptible to a second medical product in a second stage of the treatment. The kit may comprise instructions to use the medical hydrogel and the one or more sheet(s) of medical product comprising a gauze and nanofibrillar cellulose in a treatment method, such as for inducing vascularization in wounds, and/or for treating deep wounds of dermis and/or below tissue, preferably for inducing vascularization, or for other purpose disclosed herein.

For example the kit may comprise instructions to

- apply the medical hydrogel to the deep wound, for example for a time period disclosed herein,
- remove the medical hydrogel from the wound, and
- apply the medical product comprising a gauze and nanofibrillar cellulose to the wound.

The medical product comprising a gauze and nanofibrillar cellulose may comprise a gauze impregnated with nanofibrillar cellulose.

The medical hydrogels disclosed herein may be provided for use in any of the methods disclosed herein such as in a method for treating wounds, especially in methods for treating deep wounds. The method may be also a method for inducing vascularization. The subject to be treated may be a patient, such as human or animal subject or patient.

In one example a method comprises

- preferably detecting a deep wound of dermis and/or below tissue in a subject, or detecting a subject in need of treatment of a deep wound of dermis and/or below tissue,
- providing a medical hydrogel disclosed herein,
- applying the medical hydrogel to the deep wound. The medical hydrogel may be kept in the wound after application for a time period sufficient for providing vascularization in the wound or tissue(s) of the wound and/or other healing effects. The time period may be for example at least 1 day, 2 days, 3 days, 4 days, or 5 days, up to 7, 10 or 14 days, for example 1-14 days, such as 1-7 days, 1-5 days, 1-4 days, 1-3 days, 1-2 days, 2-14 days, 2-7 days, or 6-168 hours, such as for 12-168 hours or for 24-168, in some cases for 6-48 hours, such as for 6-24 hours or for 6-12 hours.

After this, *i.e.* after said time period, the medical hydrogel may be removed from the wound, and a second wound dressing may be applied to the wound. The second wound dressing may also comprise nanofibrillar cellulose, but it may be of different type. The second wound dressing may comprise a layer of nanofibrillar cellulose and preferably a layer of gauze or it may comprise a gauze impregnated with nanofibrillar cellulose. The second wound dressing may be kept in the wound for at least 6 hours or for at least 12 or 24 hours, or at least for 1 day, 2 days, 3 days, 4 days or 5 days, up to 7 days, such as for 12-168 hours, 12-72 hours, 12-48 hours or 6-24 hours. One or more further wound dressings(s) may be applied after a previous wound dressing has been removed. It is also possible to treat the wound again with similar new or unused hydrogel or with other material(s) and/or method(s) after the medical hydrogel has been removed from the wound, such as with surgical methods, for example applying a skin graft and/or suture(s).

In one example a method comprises

- preferably detecting a deep wound of dermis and/or below tissue in a subject, or detecting a subject in need of treatment of a deep wound of dermis and/or below tissue,
- providing a medical hydrogel disclosed herein,
- applying the medical hydrogel to the deep wound, preferably for a time period disclosed herein,
- removing the medical hydrogel from the wound, and
- applying a medical product comprising a gauze and nanofibrillar cellulose to the wound, such as a gauze impregnated with nanofibrillar cellulose.

The present application also provides the medical hydrogel for use in said method.

**Examples**

**Example 1**

The potential of NFC derived biomaterial, especially anionic NFC (ANFC) to provide an optimal scaffold for hASCs, and whether hASCs and ANFC promote wound healing together was studied *in vivo* in a splinted full thickness excisional skin wound model in healthy mouse before proceeding into chronic wound model. In the model, the wounds are surrounded by silicone rings attached to skin by stitches to prevent wound contraction.

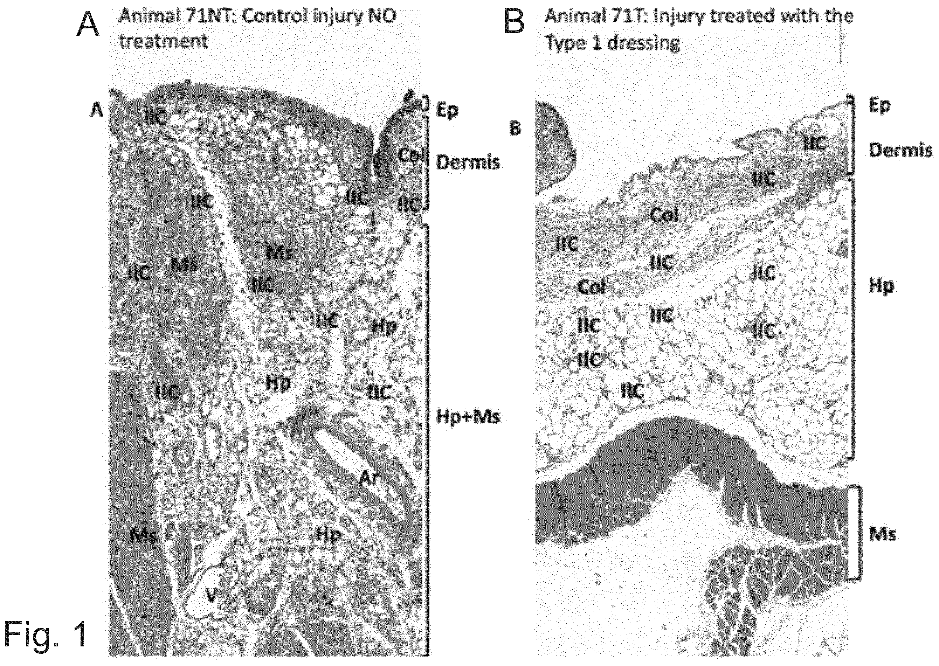

The model mimics human wound healing process, where wound repair proceeds mainly through granulation tissue formation and re-epithelialization. The aim was to avoid using additional adhesion proteins for cell transplantation. For this study, ANFC was tested together with native NFC. The safety of NFC dressing material has been proved in previous studies, while the native NFC dressing was also shown to improve the polarization of newly formed skin structures (Fig. 1)

Figure 1 shows histopathological evaluation of wounds in humanized mice treated with NFC dressing (Type 1 dressing). (A) The NOT treated Injury (Animal 71 NT): Dermis is starting to appear as small thin disorganized collagen fibers with full of inflammatory cell infiltrates. Hypodermis is present with few fat cells and also contains infiltrates of immune cells. Immune inflammatory cells are present also in the muscle layer that is disorganized, covering all the area. This indicates an early process of regeneration starting in the lower layers as expected, with acute inflammation in all the layers of the skin in regeneration. (B) Type 1 dressing treated injury (Animal 71T): Dermal layer contains collagen fibers organized in parallel, with an abundant presence of immune cells infiltration in all the dermal layer. The hypodermis has fat cells and is starting to organize with some collagen fibers, but contains still inflammatory and immune cells along recovered and organized with fat cells packed by collagen fibers, but still some blood cells are around. Muscle is recovered and packed without rests of immune cell infiltration. Ar: Artery; Col: Collagen; Ep: Epidermis; Hp: Hypodermis containing fat; IIC: Inflammatory infiltrated cells; Ms: Muscle; V: Vein.

For the wound healing experiments to study the effects of ANFC and hASCs on the healing process and the suitability of ANFC as a cell scaffold, mice were anaesthetized with isoflurane (4-5% in 5 l/min to induce, 1-2% in 1 l/min to maintain during surgery). The dorsal hair of the animals was shaved from approximately 2 cm$^2$ area on either side of the spine and the skin is disinfected with chlorhexidine. On the shaved dorsal surface, two 6 mm full thickness wounds were created using a biopsy punch, one on either side of the spine at a distance of approximately 0.5 cm from the spine and at the level of the rib cage. A silicone ring was then sutured onto the surface of the skin surrounding the excision using 8 stitches to prevent wound contraction. All reagents and materials used in the animal studies were fabricated via aseptic methods.

Mice were divided in different study groups using randomization (N = 4 per group per timepoint). The "sham" group treated with PBS serves as a control. The sample size for all groups is calculated as follows:

Chance of concluding that treatments differ when they do not (Type 1 error rate) = 5%
Co-efficient of variation, CV = 15%
Size of effect expected or of interest to detect, d = 25%

Number of replications, r = 15.7*(CV/d)$^2$, so r = 5.65 = 6.

Six wounds per treatment group were provided. Two wounds were created on each animal, therefore 4 animals are required per treatment group. There were four treatments that were analysed at 3 time points, 4 animals x 3 groups x 3 time points = 36 animals. Treatments were performed by providing healthy mice (n=36), wherein n= 12/ group x 3 groups. The animals were assigned to one or the treatment groups Sham control (n=12), ANFC (n=12) and hASC (n=12). hASCs alone was injected (100 µl, 1 x 107 cells/cm$^2$ of wound area) as a topical treatment onto the wound bed.

After the wound induction with treatments, the wounds were protected with a transparent film and gauzes, and covered by elastic bandages that bind around the animal's body. Animals were housed in a warm environment and monitored and scored daily according to the Mouse Grimace scale and the modified scoresheet as agreed with the designated veterinarian prior to commencement of the study.

The progress of wound healing was observed and photographed, and the wound healing rate calculated until wound closure. On days 3, 7 and 21, mice were anaesthetized and rectangular specimens were cut from the wounds, and divided into two halves to be used for histological study or for the preparation of a tissue protein lysate. After the operation, the animals were sacrificed.

Histological skin samples were fixed in 10% formalin for overnight at +4°C, washed, cleared in xylene and embedded in paraffin. Samples in paraffin were sectioned using a microtome and mounted on a glass slide for immunohistochemical staining. Hematoxylin and eosin (H&E) staining was used to evaluate re-epithelialization, and masson trichrome staining to evaluate granulation tissue formation. Specific marker antibodies were used for staining to study epidermal closure (keratin K14), and the expression of proliferating nuclear antigen (PCNA) to detect epidermal proliferation. Angiogenesis will be examined by staining samples with endothelial cell marker CD31 and with alpha-smooth muscle actin (a-SMA) to detect vascular networks. Infiltration of immune cells to the wound were studied with antibodies against F4.80 and Gr-1 to detect macrophages and neutrophils, respectively. Transplanted hASCs were detected with antibody against human nuclear antigen (HNA) to study their integration into the host tissue. To study paracrine secretion of GFs from hASCs in vivo, GFs, including VEGF, FGF2 and HGF were studied by double staining by appropriate antibodies with HNA to prove the bioactivity of hASCs in the host tissue.

To prepare a tissue protein lysate, a tissue sample was snap freezed in liquid nitrogen and later homogenized in RIPA buffer containing protease inhibitors and phosphatase inhibitors. Protein lysate was subjected for western blotting analysis to study the expression of GFs, including VEGF, FGF2 and HGF that were normalized to β-Actin expression to further confirm the paracrine signaling mechanisms of hASCs.

Statistical difference between two groups were assessed by Student's t-test, and comparisons between more than two groups by one-way analysis of variance (ANOVA) followed by Tukey multiple comparison test. Correlation between different data sets was analyzed using the Spearman's correlation test.

Results

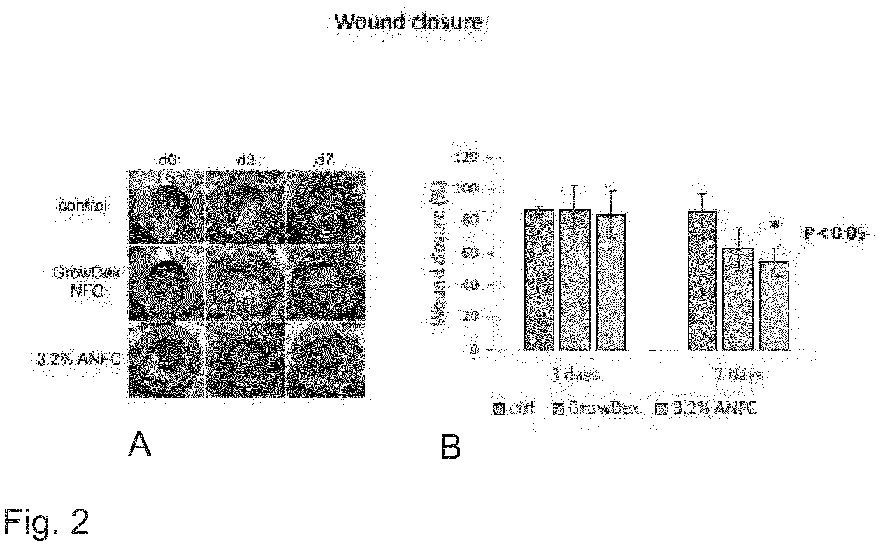

In the experiments conducted it was shown that anionic nanofibrillar cellulose (ANFC) hydrogel promotes wound dressing compared to the chemically unmodified nanofibrillar cellulose (GrowDex) and control sample (no treatment) as presented in the Figure 2. Especially the effect of the material to the pre-vascularization in the wound was studied, which is desired in deep wounds, such as wounds of dermis or below.

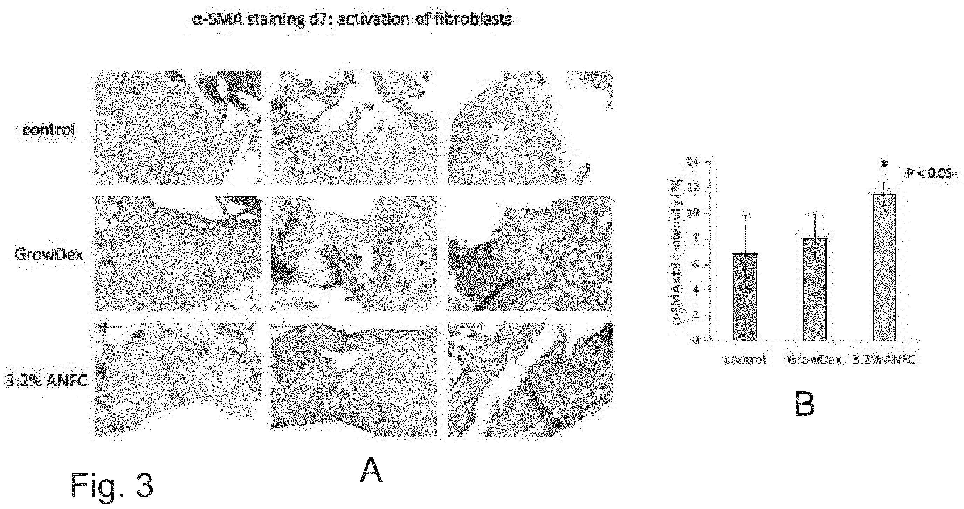

In addition to the wound dressing it was proved that ANFC hydrogel stimulates fibroblast activation compared to the control samples (Figure 3) by a-SMA staining at the 7th day (d7) of the experiments. The staining was evident with the wounds treated by 3.2% (w/w) anionic nanofibrillar cellulose, but not with chemically unmodified nanofibrillar cellulose (GrowDex). Therefore, based on these experiments, it can be concluded, that ANFC hydrogel is beneficial for fibroblast activation, and therefore results in blood vessel formation in the tissues.

Figure 3 shows results from the stimulation of the fibroblast activation. As presented in the figure, it can be seen that 3.2% (w/w) anionic nanofibrillar cellulose (ANFC) hydrogel stimulates fibroblast activation more than chemically unmodified nanofibrillar cellulose and the control sample (no treatment). Figure 3A shows $\alpha$-SMA staining d7: activation of fibroblasts in different materials. Figure 3B is a graph presenting $\alpha$-SMA stain intensity in different materials.

In comparison tests it was noticed that anionic nanofibrillar cellulose hydrogel comprising 5.3% (w/w) of nanofibrillar cellulose did not provide the vascularization effect and the hydrogel was prone to dry. However similar hydrogel comprising 2.65% (w/w) of nanofibrillar cellulose did enhance the pre-vascularization and the material remained moist. In general a percentage of about 3% (w/w) was found advantageous, and therefore most tests were carried out with a concentration of about 3.2% (w/w) of nanofibrillar cellulose.

However, the moldability of the hydrogel will decrease if the concentration of the gel is too low. The concentration of nanofibrillar cellulose below 1% (w/w) is not very useful, and especially the printability of such material when using additive manufacturing is poor. A concentration of at least 2% (w/w) is advantageous in most cases, also in respect of the additive manufacturing.

As a summary, NFC hydrogel and its derivatives, by promoting hASC cell growth and characteristics in culture, are valuable materials for a scaffold. NFC based dressing promotes the bioactivity of the host tissue to facilitate the tissue repair. By promoting wound healing, NFC hydrogel and/or dressing provide a new efficient treatment for wound healing.

**Example 2: Chicken tests**

The mouldability and stability of different hydrogels were examined by using pieces of chicken meat as a test subject. Large and deep wounds were made to the chicken, and covered with nanofibrillar cellulose hydrogels. NFC lots 11428 and 11473 were applied into the wounds.

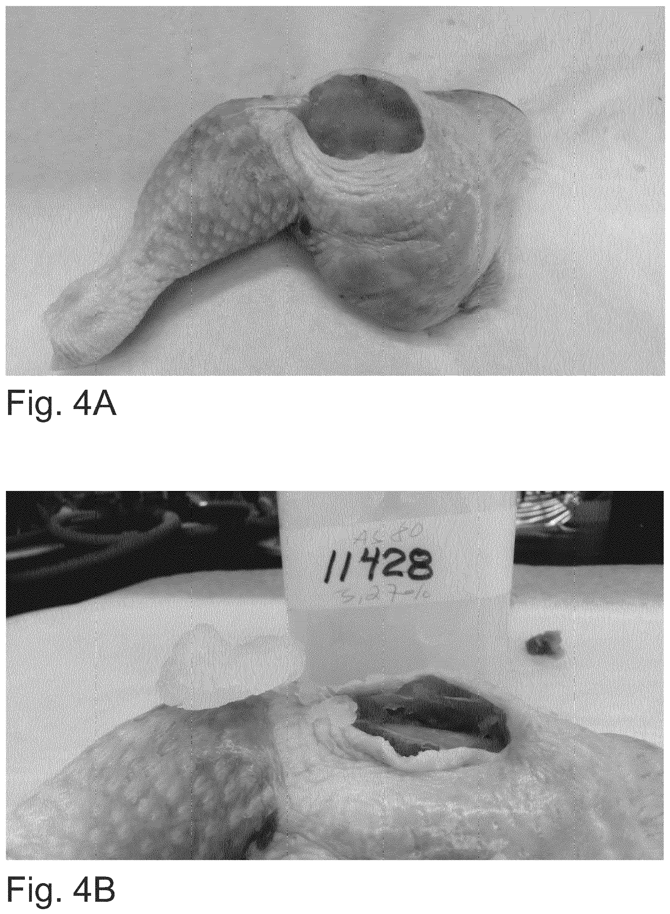

An anionic nanofibrillar cellulose hydrogel comprising 3.27% (w/w) of NFC could be moulded into a wound (Fig. 4A) by tapping manually, and it also was almost completely detached from the wound. The shape of the removed gel reproduced the shape of the wound (Figure 4B). The structure of the gel was however slightly crumbling and it left small blocks in the wound, which could be however easily rinsed off with water. This gel tolerated external water better than the 2.65% gel.

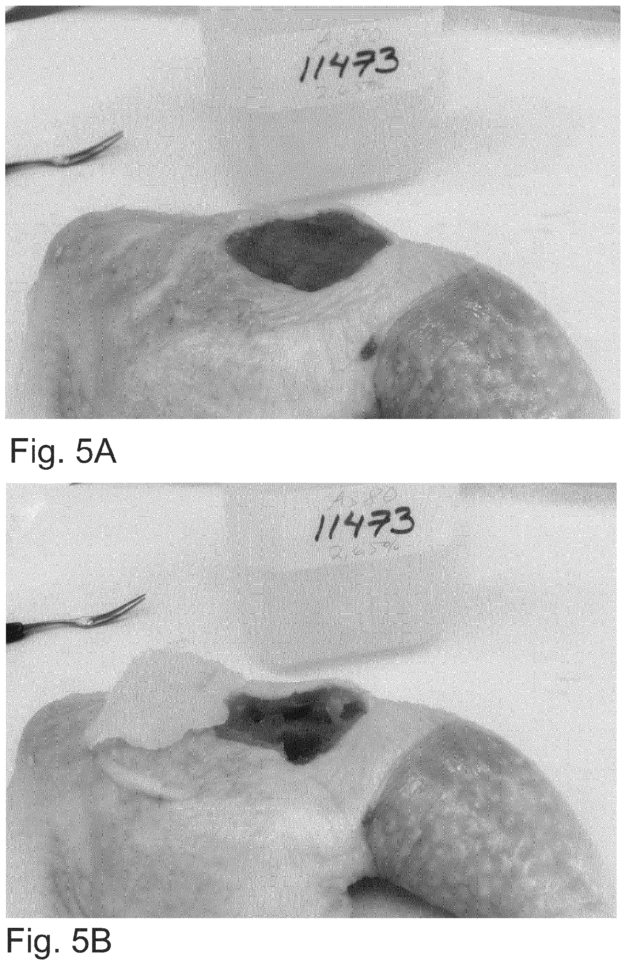

An anionic nanofibrillar cellulose hydrogel comprising 2.65% (w/w) of NFC could be moulded into a wound smoothly with very little tapping (Fig. 5A). However the gel was difficult to remove from the wound, as it was sticky and adhesive. The shape of the removed gel (Figure 5B) did not reproduce the shape of the wound as well as the 3.27% gel. The material left in the wound could be rinsed with plenty of water.

**Example 3: Measurements**

*Rheological measurements*

The rheological measurements were performed at 37°C with HAAKE Viscotester iQ Rheometer (Thermo Fisher Scientific, Karlsruhe, Germany) equipped with a Peltier system for temperature control. Results were analyzed with HAAKE RheoWin 4.0 software (Thermo Fisher Scientific). Parallel 35 mm diameter steel plate-and-plate geometry was used with a 1 mm gap in all measurements. Before each measurement, the samples were allowed to rest for 5 min at 37°C. Controlled stress amplitude sweeps were performed to determine the linear viscoelastic region for different NFC hydrogel formulations. Constant angular frequency $\omega$=1 Hz and oscillatory stress between 0.0001-500 Pa was used in all amplitude sweeps. The chosen oscillatory stresses for frequency sweeps were T=50 Pa. Shear viscosity was measured by increasing the shear rate from 0.1 to 1000 1/s.

3.2% (w/w) anionic nanofibrillar cellulose shear viscosity (at own concentration) was measured at the shear rate 0.1 1/s. The viscosity value was 1750 Pa·s.

*Water retention capacity*

Water retention capacity was measured according to the following procedure.

ÅAGWR (Åbo Akademi Gravitometric Water Retention) is a coating colour static water retention method developed by Åbo Akademi. ÅAGWR-Hydrogel is a hydrogel water retention capacity method developed for ÅAGWR device.

Liquid phase amount of the sample gone through membrane foil with certain time and pressure was measured. Absorbed water amount in hydrogel was calculated.

ÅAGWR device was used in the measurements. The device contains metal cylinders, a balancing plug, and a rubber measuring base. Whatman 17CHR blotting board and Whatman Nucleopore 0.4 $\mu$m membrane foils, diameter 47 mm were used, as well as a stopwatch and an analytical balance (accuracy 0.0001 g).

Dry content of the hydrogel was determined in oven by keeping the sample 4 hours in 105°C temperature. Weigh of the hydrogel was determined before and after drying. The blotting board was cut in 57 mm x 57 mm pieces.

The measurement procedure was as follows.

The hydrogel to be measured is completely mixed with a spoon. Pressure air is connected and adjusted to 0.5 bar as a measuring pressure of the ÅAGWR device. Two pieces of the balanced blotting board with wire side upwards are set on the rubber measuring base. A membrane foil glossy surface upwards is set on the blotting board. The metal cylinder is placed on the filter.

About 5 g hydrogel is dosed to the cylinder, and face-dressed against membrane by putting the balancing plug to the measuring cylinder for a couple of seconds. The rubber measuring base is set with filter and cylinder to the measuring table of the device and the measuring table is lifted (CYLINDER).

The rubber plug is set into place, pressure is connected on (PRESSURE) and stopwatch is started. The pressure is let to interact 3 minutes, and then the pressure is disconnected (PRESSURE), the measuring table is lift down (CYLINDER) and the rubber measuring base is taken out of the measuring table. The cylinder with membrane foils is removed from the blotting board and the blotting boards are weighed with 0.1 g accuracy.

Two parallel measurements are carried out. If test results differ over 5% from each other, more parallel measurements (max 3 measurements/sample) shall be carried out.

Calculation and reporting of results

$$X \ (g/g) = \frac{(A - B) - (a - b)}{(A \ \times \ C) \ / \ 100}$$

X    Hydrogel water retention capacity, g/g
A    Hydrogel wet weigh, g
B    Hydrogel dry weigh, g
a    Blotting board wet weigh, g
b    Blotting board dry weigh, g
C    Hydrogel dry content

Hydrogel water retention capacity test results is average of two parallel measurements. Measurement accuracy is 0.1 g/g. If test results differ over 5% from each other, more parallel measurements (max 3 measurements/sample) shall be carried out.

Water retention value 28.5 g water/g dry hydrogel was measured for 3.2% (w/w) anionic nanofibrillar cellulose hydrogel, which was found especially advantageous in the tests for applying to deep wounds and for inducing vascularization.

**Claims**

1. A method for preparing a medical hydrogel, the method comprising

    - providing anionically modified pulp, such as wood pulp, or
    - providing pulp, such as wood pulp, and modifying the pulp anionically,
    - disintegrating anionically modified the pulp until nanofibrillar cellulose having an average fibril diameter of 200 nm or less is obtained,
    - optionally homogenizing the disintegrated anionically modified pulp in one or more non-fibrillating homogenizing step, and
    - forming the nanofibrillar cellulose into a medical hydrogel having a content of nanofibrillar cellulose in the range of 1-3.5% (w/w), such as 1.1-3.5% (w/w), for example 2-3.5% (w/w).
    - preferably forming the nanofibrillar cellulose into a medical hydrogel having a viscosity in the range of 500-2200 Pa·s, such as in the range of 700-1800 Pa·s, measured with a viscometer at the concentration of the hydrogel and at 37°C at a shear rate of 0.1 1/s.

2. A medical hydrogel comprising nanofibrillar cellulose, wherein the content of nanofibrillar cellulose in the hydrogel is in the range of 1-3.5% (w/w), and the nanofibrillar cellulose comprises anionic nanofibrillar cellulose having an average fibril diameter of 200 nm or less.

3. The medical hydrogel of claim 2, wherein the content of nanofibrillar cellulose in the hydrogel is in the range of 1.1-3.5% (w/w), such as 2.0-3.5% (w/w), for example 2.5-3.5% (w/w).

4. The medical hydrogel of claim 2 or 3, wherein the hydrogel has a viscosity in the range of 500-2200 Pa·s, such as in the range of 700-1800 Pa·s, measured with a viscometer at the concentration of the hydrogel and at 37°C at a shear rate of 0.1 1/s.

5. The medical hydrogel of any of the claims 2-4, wherein the nanofibrillar cellulose has a water retention value in the range of 20-70 g water/g dry hydrogel, preferably 23-50 g water/g dry hydrogel. The water retention value may be measured with ÅAGWR (Åbo Akademi Gravitometric Water Retention) method.

6. The medical hydrogel of any of the claims 2-5, wherein the nanofibrillar cellulose, when dispersed in water, provides a zero shear viscosity in the range of 1000-50000 Pa·s, such as in the range of 2000-20000 Pa·s, and a yield stress in the range of 1-30 Pa, such as in the range of 3-15 Pa, determined by rotational rheometer at a consistency of 0.5% (w/w) by weight in aqueous medium at 22°C±1°C.

7. The medical hydrogel of any of the claims 2-6 obtained with the method of claim 1.

8. The medical hydrogel of any of the claims 2-7 for use for inducing vascularization in wounds.

9. The medical hydrogel of any of the claims 2-7 for use for treating deep wounds of dermis and/or below tissue, preferably for inducing vascularization.

10. The medical hydrogel of any of the claims 2-7 for use for treating deep wounds of dermis and/or below tissue with a method comprising

    - applying the medical hydrogel to the deep wound, preferably for at least 6 hours, 12 hours or 24 hours,
    - removing the medical hydrogel from the wound, and
    - applying a medical product comprising a gauze and nanofibrillar cellulose, to the wound, such as a gauze impregnated with nanofibrillar cellulose.

11. A method for preparing a tailored wound dressing, the method comprising

    - providing the medical hydrogel of any of the claims 2-9, preferably having a content of nanofibrillar cellulose in the range of 2-3.5% (w/w),
    - obtaining a digital three-dimensional model of an object designed to fit to a wound, and
- preparing a three-dimensional object from the medical hydrogel according to the digital three-dimensional model by additive manufacturing to obtain a wound dressing tailored to fit the wound.

12. The method of claim 11, wherein the digital three-dimensional model is obtained from a 3D scan of the wound.

13. A wound dressing tailored to fit a wound, the wound dressing comprising the medical hydrogel of any of the claims 2-10 tailored to fit to the wound, preferably obtained with the method of claim 11 or 12.

14. A kit containing the medical hydrogel of any of the claims 2-10 packed in one or more sealed package(s), or in one or more application device(s), such as a syringe, an applicator, a pump or a tube.

15. The kit of claim 14, further comprising one or more sheet(s) of medical product comprising a gauze and nanofibrillar cellulose, such as a gauze impregnated with nanofibrillar cellulose, preferably having a moisture content below 10% (w/w).

Fig. 1

Fig. 2

α-SMA staining d7: activation of fibroblasts

A

B

Fig. 3

EP 3 771 470 A1

Fig. 4A

Fig. 4B

Fig. 5A

Fig. 5B

Europäisches
Patentamt

European
Patent Office

Office européen
des brevets

# EUROPEAN SEARCH REPORT

Application Number

EP 19 39 7524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 833 540 B2 (UPM-KYMMENE CORP [FI]) 5 December 2017 (2017-12-05) * column 1, line 17 - line 27 * * column 7, line 32 - line 42 * * column 8, line 46 - line 60 * * column 9, line 38 - line 55 * * column 11, line 34 - line 37 * * column 12, line 5 - line 12 * * column 14, line 39 - column 15, line 15 * * column 17, line 7 - line 14 * ----- | 1-15 | INV. A61L15/28 A61L15/60 A61L26/00 |
| A | CHUNLIN XU ET AL: "3D printing of nanocellulose hydrogel scaffolds with tunable mechanical strength towards wound healing application", JOURNAL OF MATERIALS CHEMISTRY B, vol. 6, no. 43, 1 January 2018 (2018-01-01), pages 7066-7075, XP55657953, GB ISSN: 2050-750X, DOI: 10.1039/C8TB01757C * Abstract; page 7066 * * Materials and methods 3D-printing Post-print BDDE crosslinking; page 7067 * ----- | 11,12 | |
| A | EP 3 335 740 A1 (UPM KYMMENE CORP [FI]) 20 June 2018 (2018-06-20) * page 2, paragraphs 0001, 0005-0007 * * page 4, paragraphs 0029,0030 * * claims * ----- | 1-15 | TECHNICAL FIELDS SEARCHED (IPC) A61L |

The present search report has been drawn up for all claims

| Place of search | Date of completion of the search | Examiner |
|---|---|---|
| The Hague | 14 January 2020 | Dudás, Eszter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 19 39 7524

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2020

| Patent document cited in search report | | Publication date | Patent family member(s) | | Publication date |
|---|---|---|---|---|---|
| US 9833540 | B2 | 05-12-2017 | CA | 2897514 A1 | 28-08-2014 |
| | | | DK | 2958599 T3 | 20-02-2017 |
| | | | EP | 2958599 A1 | 30-12-2015 |
| | | | ES | 2614908 T3 | 02-06-2017 |
| | | | FI | 126109 B | 30-06-2016 |
| | | | US | 2015367024 A1 | 24-12-2015 |
| | | | WO | 2014128354 A1 | 28-08-2014 |
| EP 3335740 | A1 | 20-06-2018 | CN | 110072567 A | 30-07-2019 |
| | | | EP | 3335740 A1 | 20-06-2018 |
| | | | US | 2019336643 A1 | 07-11-2019 |
| | | | WO | 2018109275 A1 | 21-06-2018 |

EPO FORM P0459

For more details about this annex : see Official Journal of the European Patent Office, No. 12/82